# EUROPEAN PATENT SPECIFICATION

(11) **EP 2 481 794 B1**
(45) Date of publication and mention of the grant of the patent: **23.08.2017**
(21) Application number: 10015106.7
(22) Date of filing: 29.11.2010
(51) Int. Cl.: C12N 5/00, C12M 1/12

(54) **Patterned substrates for cell applications**
Strukturierte Substrate für Zellanwendungen
Substrats à motifs pour applications cellulaires

(43) Date of publication of application: 01.08.2012
(73) Proprietor: Karlsruher Institut für Technologie, 76131 Karlsruhe (DE)
(72) Inventor: Levkin, Pavel, 76344 Eggenstein-Leopoldshafen (DE); Geyer, Florian, 69121 Heidelberg (DE); Liebel, Urban, 69234 Dielheim Horrenberg (DE)
(74) Representative: Müller-Boré & Partner Patentanwälte PartG mbB

(56) References cited:
- WO-A2-2009/137267
- US-A1- 2005 279 730
- HAN YEHUA ET AL: "Monolithic superhydrophobic polymer layer with photopatterned virtual channel for the separation of peptides using two-dimensional thin layer chromatography-desorption electrospray ionization mass spectrometry.", ANALYTICAL CHEMISTRY 15 MAR 2010 LNKD- PUBMED:20151661, vol. 82, no. 6, 15 March 2010 (2010-03-15) , pages 2520-2528, XP002634428, ISSN: 1520-6882
- ZHANG XINTONG ET AL: "A transparent and photo-patternable superhydrophobic film.", CHEMICAL COMMUNICATIONS (CAMBRIDGE, ENGLAND) 14 DEC 2007 LNKD- PUBMED:18361379, no. 46, 14 December 2007 (2007-12-14), pages 4949-4951, XP002634429, ISSN: 1359-7345
- HORAK D ET AL: "Poly(2-hydroxyethyl methacrylate)-based slabs as a mouse embryonic stem cell support", BIOMATERIALS, ELSEVIER SCIENCE PUBLISHERS BV., BARKING, GB, vol. 25, no. 22, 1 October 2004 (2004-10-01), pages 5249-5260, XP004504216, ISSN: 0142-9612, DOI: DOI:10.1016/J.BIOMATERIALS.2003.12.031
- ZAHNER DAVID ET AL: "A facile approach to superhydrophilic-superhydrophobic patterns in porous polymer films.", ADVANCED MATERIALS (DEERFIELD BEACH, FLA.) 19 JUL 2011, vol. 23, no. 27, 19 July 2011 (2011-07-19) , pages 3030-3034, ISSN: 1521-4095

## Description

The present invention relates to a patterned substrate with a transparent solid support coated with a porous polymer layer or film comprising hydrophilic areas surrounded by hydrophobic areas separating the hydrophilic areas into a predetermined spatial pattern, a method for the manufacture of said patterned substrate, a method for creating predefined patterns of cells by cultivating them on said patterned substrate, a method for cultivating cells on said patterned substrate, and a use of said patterned substrate for cultivating cells.

Microarrays and particularly cell arrays are currently needed for screening of large biomolecule-libraries, such as RNAs, DNAs, proteins and small molecules with respect to their biological functions and for fundamental investigation of cell and gene-functions. Many research facilities both in academia and in industry need advanced high-density arrays to improve their screening-efficiency, velocity and quality. Many screens will first become possible or significantly more affordable with the development of next generation microarrays and cell arrays, respectively. The whole field is developing quickly and a potent new technology can turn into large-scale applied routine in very short time.

In 2007 Zhang et al. published a method to produce a superhydrophobic / superhydrophilic micropattern on a thin TiO₂ film.[7] They used a spin-coated TiO₂ film with a thickness of 500 nm. The production process formed a hierarchical nano and micro-scaled roughness. The roughness was a result of the fast evaporation of the solvent during the spincoating process. The film was subsequently treated with fluoroalkylsilane (FAS) by chemical vapor deposition. This resulted in an increase in contact angle from ∼ 0° (pure TiO₂) up to 165°. The photocatalytical deposition of organic compounds on TiO₂ surfaces was used for patterning the superhydrophobic surface. It was covered with a photomask and irradiated with UV-light. This allowed the formation of an array with square shaped spots with a minimal side length of 5 µm.

Although the photocatalytical decomposition of organic molecules on TiO₂ can easily be used to photograft a pattern into the FAS-monolayer, it also renders the pattern highly instable against UV-irradiation. Long storage even under artificial light will be impossible for the array itself and preprinted biomolecules will be subsequently degraded. Especially large, complex molecules like RNAs or DNAs can be easily damaged. Even worse will be the effect of microscopic imaging, what often uses fluorescence marked cells or biomolecules. The intense fluorescent light could possibly initiate the catalytic degradation of the array and its biomolecules.

In 2009 Reymann et al. published the layout of a lithographically produced array for reverse transfection screens.[8] It currently represents perhaps the most advanced cell array and consists of 9216 microspots that are etched in a thin titanium coating of 200 nm on an about 12 x 8 cm glass plate with a thickness of 500 µm. Spots are circles with a diameter of 400 µm and with either 1125 µm or 750 µm spot-to-spot distance. The micro-wells are formed by etching the titanium layer. The etched cavities are used as micro-wells that can be filled with iRNA transfection mixtures.

The combination of the well-format with a flat topography eliminated optical problems, allowed a great gain in spot-density and prevented cross-contamination between adjacent spots, but the 200 nm deep physical barriers are not enough to prevent cell-migration (a typical cell has a height of several µm). This will lead to severe systematical errors if the spot-density is pushed towards the geometrical limit. Spot geometry itself can be controlled only to some degree as the production does not allow controlling the wettability of the surface. The spots are circular thus preventing an ideal packing. Another problem is the high cost and the advanced equipment needed for the production of the arrays using the lithographic approach.

In a most recent example, Neumann et al. carried out an iRNA based full genome scan using several cell arrays. The authors identified more than 500 genes linked to the cell mitosis, more than half of them providing a novel functional link to cell division.[4] This example shows the great potential for the use of cell arrays with increasing density. On the other hand, the work of Neumann et al. also uncovers current limitations of the technology and reveals requirements for the further development of cell arrays.

In this context, Zhang *et al.* (Zhang, X. et al.; Chem Commun (Camb), 46; 2007; pp. 4949-4951) describes a transparent and photo-patternable superhydrophobic TiO₂ film prepared by spin-coating a TiO₂ slurry on a glass substrate and modifying the resultant film with fluoroalkylsilane molecules and patterning by illumination with UV light.

Any microarray and cell array, respectively, should also have sufficient transparency and a flat topography.[1,5] Another important criterion is a low fabrication price of the chip. The spot geometry should be square in order to achieve dense packing of the spots and to ease the readout. In case of the genome-on-a-chip cell array the minimal number of spots reaches about 50000 (21000 genes from the human genome plus control and double check experiments). This array should fit onto a customary microtiter scaled plate (12 x 8 cm) to ensure the usability of conventional microplate handling robots and microscopes. The availability of such genome-on-a-chip microarray would make full-genome screens significantly less expensive and time consuming and could turn the genome-wide reverse transfection experiments into a routine method available to most of the biological laboratories.

However, several technological challenges have so far prevented the development of a genome-on-a-chip cell array. The first difficulty is, that cross contamination becomes a serious problem with increasing spot density and thus, decreasing the distance between spots. Secondly, high density packing of the spots requires non-circular (e.g. square) geometry of the spots, which sets additional constraints on the ability to control surface chemistry. The spot size depends, *inter alia,* on the surface tension of spotting solutions that may differ from one probe-molecule-cocktail to another. This may lead to the variation of the spot size and therefore limits the minimum distance between spots. Another serious problem, arising from the high proximity of spots in a high-density cell array, is the migration of transfected cells between adjacent spots that causes growing systematical errors with smaller spot-to-spot distance.[2]

Thus, the problem underlying the present invention is to provide new means for an efficient cultivation of cells on a patterned substrate which overcome the shortcomings of the protocols known in the art. In particular, the problem underlying the present invention is to provide a patterned substrate and cell array, respectively, capable to prevent cross contamination between adjacent spots, to eliminate or at least to keep to a minimum cell migration between adjacent spots, which allows high data-quality and investigation with common screening techniques, and which is biocompatible, mechanically stable and does not degrade within a reasonable time-span. In addition, it is important to be able to precisely control spot geometry, size and density.

The solution to the above technical problem is achieved by the embodiments characterized in the claims.

In particular, the present invention relates to a patterned substrate with a solid support coated with a porous polymer layer or film comprising
(i) hydrophilic areas; surrounded by
(ii) hydrophobic areas separating the hydrophilic areas into a predetermined spatial pattern;
wherein the porous polymer layer or film comprises porous poly(2-hydroxyethyl methacrylate-co-ethylene dimethacrylate) (HEMAcoEDMA) which has been copolymerized in the presence of 40 to 90 vol% of an inert porogen,
wherein the porous polymer layer or film has a thickness of 15 µm or less, and wherein the hydrophobic areas are created by modifying the porous polymer layer or film.

In the following the terms "spot" and "area" will be used synonymously. Preferred embodiments identified for a "spot" are also valid for an "area" and vice versa. In the following the terms "hydrophilic" and "superwetting" will be used synonymously.

The hydrophobic, preferably superhydrophobic, surfaces and barriers, respectively, of the patterned substrate according to the present invention result in the prevention of proliferation and migration of cells. The use of such hydrophobic, preferably superhydrophobic, barriers is a completely novel approach for controlling cell proliferation and migration. The combination of the watertight hydrophilic (superwettable) areas with the highly efficient cell-barriers represents a new approach for designing e.g. cell arrays and has the potential to push the spot-density towards its theoretical maximum that is only given by the necessary amount of cells in each spot. Stringent control over all important parameters (feature shape and size, absorbable volume, thickness of the polymer film, cell-migration, transparency) and the simple and inexpensive production enables an easy adjustment to any other application where cell-growth in a predetermined spatial order on a flat substrate is needed.

In general, surface properties can be classified into hydrophobic and hydrophilic surfaces depending on the value of the water contact angle (WCA). A surface having a WCA greater than 90° is referred as hydrophobic, whereas a WCA smaller than 90° is referred as hydrophilic. Maximum water contact angle on a smooth surface is about 120°. In practice, two types of WCA values are used: static and dynamic. Static water contact angles (θₛₜₐₜ) are obtained by sessile drop measurements, where a drop is deposited on the surface and the value is obtained by a goniometer or a specialized software. Dynamic contact angles are non-equilibrium contact angles and are measured during the growth (advancing WCA θ_{adv}) and shrinkage (receding WCA θ_{rec}) of a water droplet. The difference between θ_{adv} and θ_{rec} is defined as contact angle hysteresis (CAH). In a preferred embodiment of the present invention, surfaces with high WCA, preferably greater than 130°, more preferably greater than 140°, most preferably greater than 150°, and/or low water CAH (less than about 10°) are called superhydrophobic. Water droplets do not stick to such surfaces and simply roll off. In a preferred embodiment of the present invention, surfaces with WCA less than 20°, preferably less than 10°, more preferably less than 5°, most preferably close to or of 0° are called superhydrophilic. Water droplets are rapidly absorbed by such surfaces.

In a preferred embodiment of the present invention, the patterned substrate is a microarray, more preferably a cell microarray. In another preferred embodiment of the present invention, the patterned substrate has a flat surface.

In another preferred embodiment of the present invention, the patterned substrate is a microplate having hydrophilic, preferably superhydrophilic, patterns on a hydrophobic, preferably superhydrophobic, background. In a more preferred embodiment of the present invention, the patterned substrate is a microplate having 6, 12, 24, 96, 384, 1536 hydrophilic, preferably superhydrophilic, areas on a hydrophobic, preferably superhydrophobic, background.

Superhydrophobic and superhydrophilic materials are known in the art [10]. According to the present invention, the porous polymer layer or film comprises porous poly(2-hydroxyethyl methacrylate-co-ethylene dimethacrylate) (HEMAcoEDMA) which has been copolymerized in the presence of 40 to 90 vol% of an inert porogen.

In a preferred embodiment of the patterned substrate according to the present invention, the pattern is created by modifying the surface of the porous polymer layer using photografting through a photomask. In a more preferred embodiment of the present invention, the hydrophobic areas are created by photografting the porous polymer layer being a porous hydrophilic layer.

In a preferred embodiment of the present invention, the patterned substrate is a microarray which preferably exhibits an array-pattern which is created by photografting the porous polymer layer using a photomask. The use of photochemical methods allows precisely controlling the geometry, size and distance between the spots by the design of the photomask.

According to the present invention, the porous polymer layer itself is hydrophilic, preferably superhydrophilic. In such an embodiment, the hydrophobic, preferably superhydrophobic, barriers are created by photografting the porous polymer layer.

According to the present invention, the hydrophobic, preferably superhydrophobic, barriers are preferably created by modifying, preferably photografting the porous polymer layer being a porous (super)hydrophilic polymer layer. In a preferred embodiment of the present invention, the hydrophobic, preferably superhydrophobic, barriers are obtained by UV-initiated photografting the porous polymer using a compound capable to impart a hydrophobic, preferably superhydrophobic, functionality to the hydrophilic, preferably superhydrophilic, porous polymer. In a preferred embodiment of the present invention, the hydrophilic, preferably superhydrophilic, porous polymer layer is photografted using a photografting mixture containing one or more mono- or poly- vinyl monomers selected from the group consisting of alkyl acrylates, alkyl methacrylates, aryl acrylates, aryl methacrylates, aryl alkyl acrylates, aryl alkyl methacrylates, fluorinated alkyl acrylates, fluorinated alkyl methacrylates, for example 2,2,3,3,3-pentafluoropropyl methacrylate, styrene, vinylnaphthalene, vinylanthracene, alkylene diacrylates, alkylene dimethacrylates, alkylene diacrylamides, alkylene dimethacrylamides, hydroxyalkylene diacrylates, hydroxyalkylene dimethacrylates, wherein the alkylene group consists of 1-4 carbon atoms, oligoethylene glycol diacrylates, vinyl esters of polycarboxylic acids, pentaerythritol diacrylate, pentaerythritol triacrylate, pentaerythritol dimethacrylate, and pentaerythritol trimethacrylate and derivatives thereof, wherein the alkyl group in each of the alkyl monomers has 1-18 carbon atoms. Particularly preferred is a photografting mixture consisting of 15wt.% 2,2,3,3,3-pentafluoropropyl methacrylate, 1wt.% ethylene dimethacrylate in 1/3 (v./v.) mixture of water - tert-butanol containing 0.25wt.% benzophenon.

In the thus obtained pattern, an easy and homogeneous adsorption of the spotting solution in the porous hydrophilic areas can be guaranteed. Each area is separated from adjacent areas with hydrophobic, preferably superhydrophobic, barriers that render the areas impermeable for all water based solutions.

In a preferred embodiment of the present invention, the hydrophilic areas of the patterned substrate are separated by the hydrophobic areas so as to form an array of hydrophilic areas surrounded by a hydrophobic porous polymer surface.

In another preferred embodiment of the patterned substance according to the present invention, the hydrophilic areas are separated by the hydrophobic, preferably superhydrophobic, areas of porous polymer surface so as to form hydrophilic, preferably superhydrophilic, channels. In a more preferred embodiment of the present invention, the patterned substance comprises hydrophilic, preferably superhydrophilic, channels on a hydrophobic, preferably superhydrophobic, background and the patterned substance is used for microfluidic applications.

In another preferred embodiment of the present invention, the hydrophilic, preferably superhydrophilic, areas which are separated by the hydrophobic, preferably superhydrophobic, barriers in the patterned substrate have a square shape. Hydrophilic, preferably superhydrophilic, areas having a square shape allow dense packing of the areas and ease the readout. Nevertheless, the patterned substrate according to the present invention is not limited to any array-format, but represents a more general and novel approach that allows patterning and for example culturing of cells in a predesigned spatial order.

In one embodiment of the present invention, the area-density of the hydrophilic, preferably superhydrophilic, areas is increased. Preferably, the amount of hydrophilic, preferably superhydrophilic, areas is at least 200 per cm² of the patterned substrate, more preferably at least 300 per cm² of the patterned substrate, even more preferably at least 400 per cm² of the patterned substrate. In a particularly preferred embodiment of the present invention, the amount of hydrophilic, preferably superhydrophilic, areas is at least 500 per cm² of the patterned substrate.

According to an even more preferred embodiment of the present invention, the patterned substrate of the present invention has a size which fits on a standard microtiter scaled plate (12 x 8 cm), preferably a size of about 11 x 7 cm with at least 40000, preferably at least 50000 hydrophilic areas separated by hydrophobic, preferably superhydrophobic, background.

Preferably, in the patterned substrate according to the present invention, the overall area-number on a single chip is significantly increased. When used as a cell microarray, minimal area of a single area in the patterned substrate according to the present invention is limited by the amount of cells needed in each area to obtain statistically valid data.[4,5] In a particularly preferred embodiment of the present invention, each of the hydrophilic, preferably superhydrophilic, areas defined in (i) has a side length of 1000 µm or less, preferably 600 µm or less, more preferably 400 µm or less, and most preferably 335 µm or less, particularly under the provision that the hydrophilic, preferably superhydrophilic, areas have a square shape. In another preferred embodiment of the present invention, the size of the hydrophilic, preferably superhydrophilic, areas is large enough to accommodate at least 100 cells, preferably 200 cells, more preferably at least 300 cells.

In a preferred embodiment of the present application the hydrophobic, preferably superhydrophobic, barriers have a width of 500 µm or less, preferably 200 µm or less, more preferably 100 µm or less and even more preferably 60 µm or less. A significant advantage of the patterned substrate according to the present invention is that the hydrophobic, preferably superhydrophobic, barriers completely prevent cross-contamination between the areas, despite the reduced width of the barriers.

Further, according to the present invention, the thickness of the porous polymer layer is 15 µm or less. Regarding transparency properties or reducing manufacturing costs, a thin porous polymer layer is preferred.

The patterned substrate according to the present invention preferably further comprises (iii) at least one biologically active agent provided in at least one hydrophilic, preferably superhydrophilic, area. According to a preferred embodiment, each hydrophilic, preferably superhydrophilic, area of the patterned substrate is provided with at least one biologically active agent. In this embodiment, the hydrophobic, preferably superhydrophobic, barriers not only prevent cross-contamination of any aqueous liquids between the areas, but also prevent proliferation and migration of cells between hydrophilic areas. That is, hydrophobic, preferably superhydrophobic, barriers between the hydrophilic, preferably superhydrophilic, areas of the patterned substrate according to the present invention stop cell proliferation and migration from one area to another. With this technique, a patterned substrate can be designed which area-density is not achievable with common methods.

The patterned substrate according to the present invention, preferably a flat surface patterned substrate, has no physical barriers between the areas, thus permitting parallel cell seeding which is very important for achieving high data-quality.[2] The absence of physical barriers also eliminates optical problems that occur with further miniaturization of microwell plates. The high density of the microspots can accelerate and simplify data acquisition. Another important advantage is that much smaller amounts of probe-molecules are required for single transfection experiments, thus making the method significantly less expensive than the liquid-phase analogue. Cost is an important factor in many screens as large biomolecule-libraries like iRNA-libraries are very expensive.

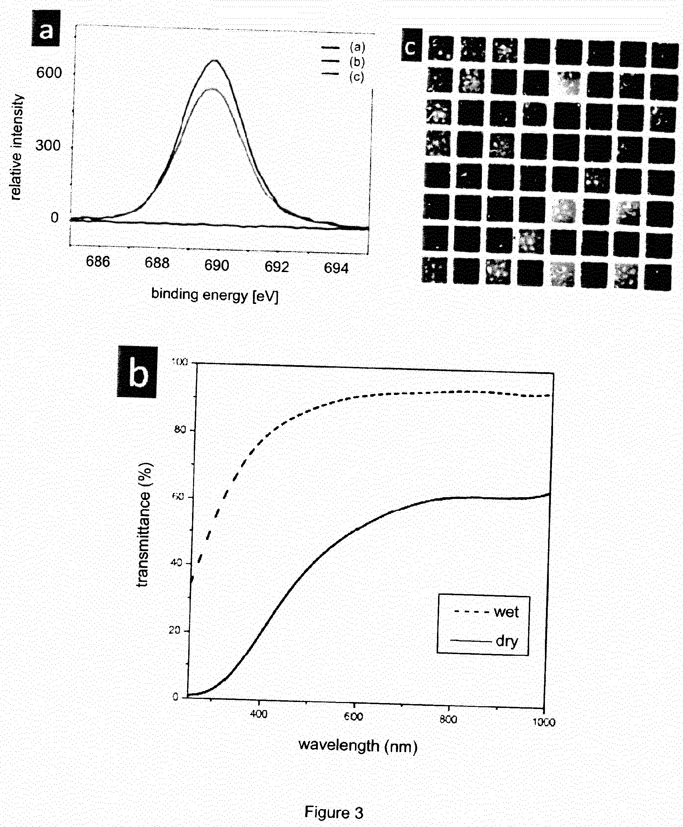

On the other hand, the very good wetting property of the porous polymer surface inside hydrophilic areas guarantees an easy and homogeneous spreading of the solutions inside the areas independently of their geometry. In a preferred embodiment of the present invention, each single hydrophilic, preferably superhydrophilic, area is filled with a water solution of biomolecules that are required for different cell experiments, preferably selected from the group, consisting of RNAi screening components, DNAs, proteins, drugs or drug candidates, peptides, recombinant proteins and other small- and macro-molecule libraries. Figure 3c visualizes an example of the watertight barriers and homogeneous spreading of spotting solutions.

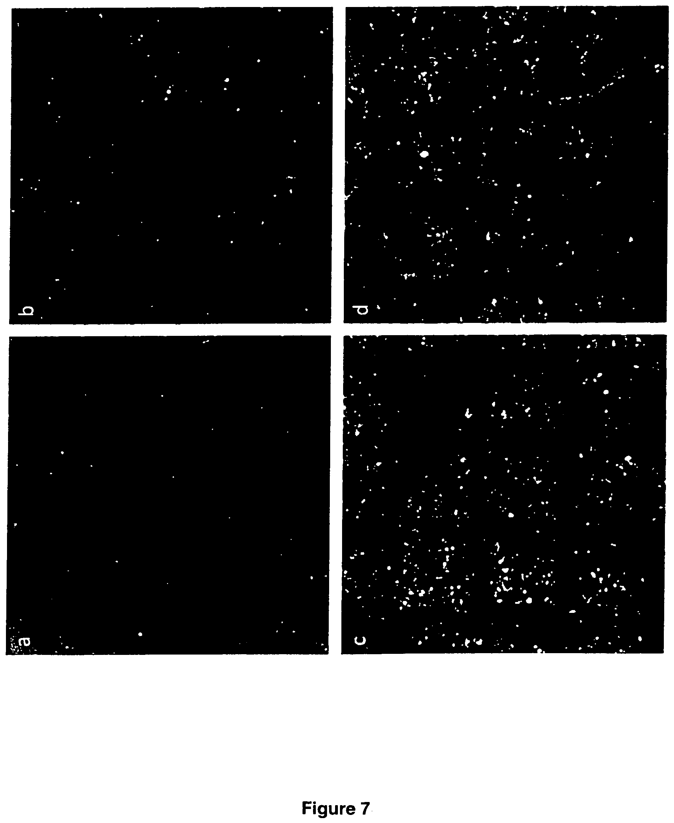

In addition, the hydrophobic, preferably superhydrophobic, barriers prevent cells from migrating between the adjacent areas. Thus, the serious problem of cell-migration between the areas known for cell arrays of the prior art can be solved using the patterned substrate according to the present invention. Figure 7 shows an example of the cell-resistant property of the barrier material. In a preferred embodiment of the present application a full genome wide RNAi screen using a single standard sized (12 x 8 cm) microplate is performed.

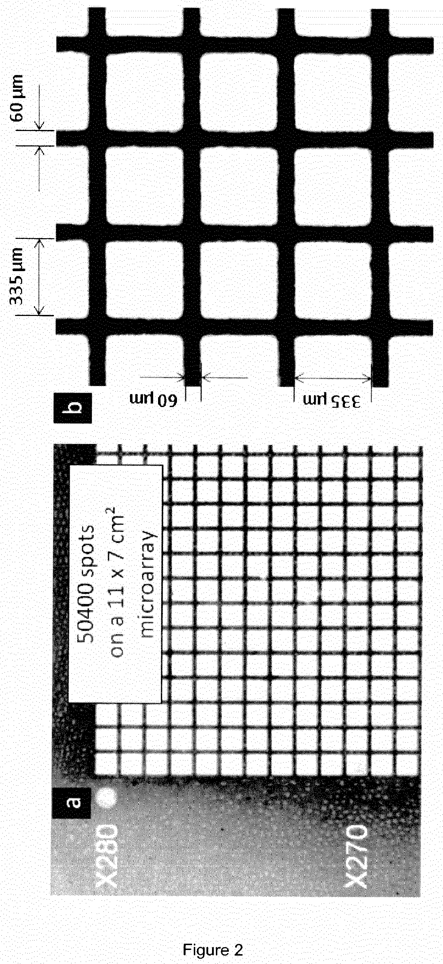

An example of the design of an array with the capacity for a full genome application is shown in Fig. 2. In a preferred embodiment of the present invention, on the usable area of a standard microtiter plate (7 x 11 cm²) 50400 areas (180 x 280 areas) are accomodated. In another preferred embodiment of the present invention, every square-shaped area has a side length of approximately 335 µm and/or the areas are divided by 60 µm wide barriers.

The term "biologically active agent" as defined herein refers to any substance which does not prevent any biological tests carried out in the hydrophilic, preferably superhydrophilic, areas. The biologically active agent may be an agent selected from the group consisting of nucleic acid molecules, like plasmids, DNA, RNA, iRNA, small molecules, macromolecules, drugs, proteins, peptides, tissue samples, polymers, hydrogels, particles, nanoparticles such as metal nanoparticles, semiconductor quantum dots, metal, carbon or polymer nanotubes and combinations thereof. In another embodiment, a biologically active agent as defined herein may be a substance suitable for binding a biomolecule or a cell to the hydrophilic, preferably superhydrophilic, area, like for example an antibody or a fragment thereof or a ligand or a biomolecule. In a particularly preferred embodiment of the present application, the biologically active agent is an antibody or a fragment thereof or a ligand binding to a surface molecule of a cell as defined herein.

In another preferred embodiment of the present application, a biologically active agent is a nucleic acid, more preferably a nucleic acid molecule, most preferably a nucleic acid molecule being selected from the group consisting of DNA, RNA, plasmids and iRNA agents. In a particularly preferred embodiment of the present application, the biologically active agent is a nucleic acid molecule and at least one cell as defined herein is transfected with the nucleic acid prior to cultivation in the hydrophilic, preferably superhydrophilic, area. In a more particularly preferred embodiment of the present invention, the array of the present invention has a size which fits on a standard microtiter scaled plate (12 x 8 cm) with at least 10000, preferably at least 20000, 30000, 40000, preferably at least 50000 hydrophilic, preferably superhydrophilic, areas and a full iRNA-library plus all needed control-experiment and double checks are pre-printed in the at least 40000, preferably at least 50000 hydrophilic, preferably superhydrophilic, areas. In another preferred embodiment of the present invention, the patterned substrate is a genome-on-a-chip cell array. In a more particularly preferred embodiment of the present invention, the patterned substrate of the present invention has a size which fits on a standard microtiter scaled plate with at least 40000, preferably at least 50000 hydrophilic, preferably superhydrophilic, areas and the 21000 iRNAs, corresponding to the 21000 genes from the human genome, are pre-printed into hydrophilic areas. The need for additional control and double check experiments results in the at least 40000, preferably at least 50000 hydrophilic, preferably superhydrophilic, areas.

The pattern of the biologically active agents can be easily adjusted as only the photomask has to be changed. This adjustment is inexpensive and can be done in short time, so the technique is also applicable to small-scaled individualized biological and biochemical research.

The present invention further provides a method for the manufacture of the patterned substrate according to the present invention, comprising the steps of:
(a) providing a polymerization mixture between two solid supports, wherein the polymerization mixture comprises 2-hydroxyethyl methacrylate (HEMA) and ethylene dimethacrylate (EDMA) in the presence of 40 to 90 vol% of an inert porogen;
(b) polymerizing the polymerization mixture by free radical polymerization to form a porous polymer layer having a thickness of 15 µm or less;
(c) removing one of the two solid supports; and
(d) modifying the surface of the porous polymer layer, thereby creating a pattern of hydrophilic areas surrounded by hydrophobic areas.

The solid support may be of any material known in the art which is solid, preferably at a temperature within the range of -30°C to 130°C, more preferably within the range of 0°C to 40°C, more preferably at room temperature. According to the present invention, the solid support can be selected from the group consisting of glass, metal, such as a stainless steel plate, or an aluminum foil, plastic, concrete, wood, and masonry. In a preferred embodiment of the present invention, the solid support is transparent, and in a particularly preferred embodiment of the present invention, the solid support is a glass solid support. Such a glass solid support may be activated and/or modified prior to use.

According to a preferred embodiment of the present invention, in order to achieve covalent attachment of the porous polymer layer, the glass surface is preferably first activated, and than functionalized with an anchor-group. For example, cleaned glass plates may be immersed in 1 M NaOH for one hour and afterwards washed with deionized water followed by immersing them in 1 M HCl for 30 min, washing with deionized water and drying with a nitrogen gun. Further, the glass solid support according to the present invention may be modified by for example dropping several drops of a solution containing 20% 3-(trimethoxysilyl)propyl methacrylate in ethanol (adjusted to pH = 5 with acetic acid) on an activated glass plate, covering the glass plate with another activated glass plate, thereby ensuring that no bubbles are trapped between the two glass plates, reapplying said solution after 30 minutes and after another 30 minutes washing the glass plates with acetone and drying with a nitrogen gun.

Within the scope of the present invention, the glass solid supports may be made inert for example by fluorination using for example tridecafluor-(1,1,2,2)-tetrahydrooctyltrichlorosilane placed in a vacuumated dessicator together with the glass solid support for 2h.

In step (a) of the method for the manufacture of a patterned substrate according to the present invention a polymerization mixture is provided between two solid supports. The solid supports may be made of the same material, or of different materials. In particular, the aforementioned materials suitable for the patterned substrate according to the present invention are preferred. In a particularly preferred embodiment of the present invention, the solid supports are made of a glass material, of which one or both may be modified and/or activated prior to use, such as a glass modified with anchor groups. In a preferred embodiment of the present invention, the solid support is a solid support as defined herein.

In step (b) of the method for the manufacture of a patterned substrate according to the present invention polymerization is carried out in the mold between the two solid supports. The polymerization involves a free radical polymerization of a polyvinyl monomer and a monovinyl monomer in the presence of porogenic solvents as defined above. The free radical initiation is preferably done either thermally or by irradiation with UV- or visible light or γ-irradiation. When polymerization is carried out by thermal initiation, the thermal initiator is generally a peroxide, a hydroperoxide, or an azo-compound selected from the group consisting of benzoyl peroxide, potassium peroxodisulfate, ammonium peroxodisulfate, *t*-butyl hydroperoxide, 2,2'-azobisiobutyronitrile (AIBN), and azobisisocyanobutyric acid, and the thermally induced polymerization is performed by heating the polymerization mixture to temperatures between 30°C and 120°C. Polymerization can also be achieved using photoinitators including, but not limited to, benzophenone, 2,2-dimethoxy-2-phenylaceto-phenone, dimethoxyacetophenone, xanthone, thioxanthone, camphorquinone their derivatives, and mixtures thereof.

According to the present invention, the porous polymer layer possesses bulk hydrophilicity and is prepared by radical polymerization of a mixture comprising 2-hydroxyethyl methacrylate (HEMA) and ethylene dimethacrylate (EDMA) in the presence of 40 to 90 vol% of an inert porogen.

The porogen (*i.e.* liquids that cause formation of the porous structure of the polymer) used to prepare the porous polymer layer in accordance with the present invention may be selected from a variety of different types of compounds. For example, suitable liquid porogens include aliphatic hydrocarbons, aromatic hydrocarbons, esters, amides, alcohols, ketones, ethers, solutions of soluble polymers, and mixtures thereof. For preparation of hydrophilic, preferably superhydrophilic, materials, water may also be used. The porogen is generally present in the polymerization mixture in an amount of from about 40 to 90 vol%, more preferably from about 50 to 80 vol%. In a preferred embodiment, the porogen is a mixture of 1- decanol and cyclohexanol.

In a preferred embodiment of the method for the manufacture of the patterned substrate according to the present invention, the inert porogen is selected from the group consisting of aliphatic hydrocarbons, aromatic hydrocarbons, esters, amides, alcohols, ketones, ethers, solutions of soluble polymer, water, and mixtures thereof.

According to the present invention, the porous polymer layer comprises porous poly(2-hydroxyethyl methacrylate-co-ethylene dimethacrylate) (HEMAcoEDMA).

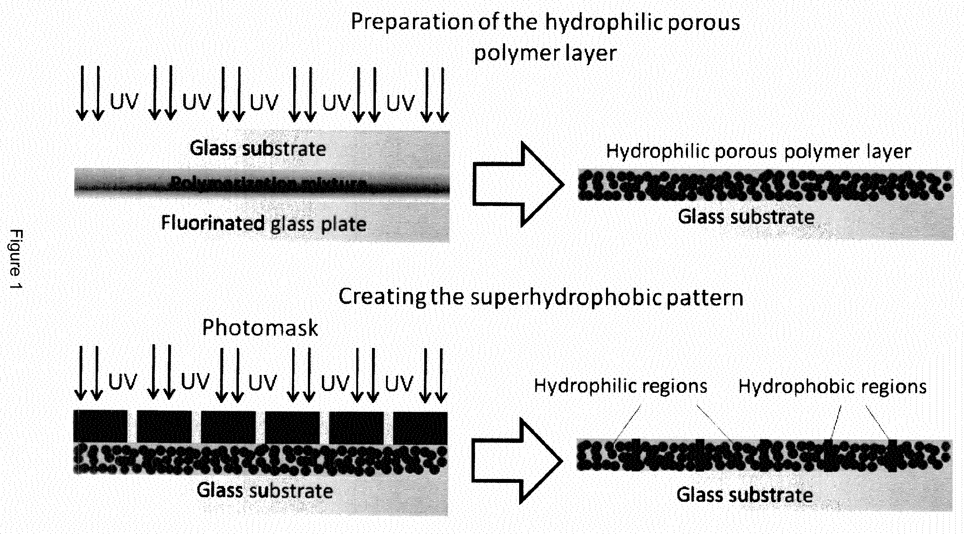

According to the present invention, the hydrophilic porous polymer layer is made of poly(2-hydroxyethyl methacrylate-co-ethylene dimethacrylate) synthesized by copolymerization of 2-hydroxyethyl methacrylate (HEMA) and ethylene dimethacrylate (EDMA) in the presence of porogens (as shown for example in Fig. 1).

According to the present invention, the ratio of polyvinyl monomer to monovinyl monomer is not limited as long as the obtained porous polymer exhibits hydrophilic, preferably superhydrophilic, properties. In a preferred embodiment, the polyvinyl monomer content is within the range of 10 to 50wt.%, preferably 15 to 45wt.%, more preferably 20 to 40wt.%, based on the total amount of the polymerization mixture. The monovinyl monomer content is preferably within the range of 5 to 30wt.%, preferably 10 to 25wt.%, more preferably 15 to 20wt.%, based on the total amount of the polymerization mixture.

After completion of the polymerization, one of the two substrates is removed in step (c).

In step (d) of the method for the manufacture of a patterned substrate according to the present invention the surface of the porous polymer layer is modified, thereby creating a pattern of hydrophilic areas surrounded by hydrophobic areas. In a preferred embodiment of the present invention, the modification is carried out as described herein, most preferably by photografting including the various embodiments for photografting as described herein.

In a preferred embodiment of the method for the manufacture of the patterned substrate according to the present invention, the hydrophobic pattern is produced by photografting of the hydrophilic porous polymer layer with hydrophobic mono- or poly-vinyl monomers or mixtures thereof.

In a more preferred embodiment of the method for the manufacture of the patterned substrate according to the present invention, the hydrophobic pattern is produced by photografting of the hydrophilic porous polymer layer with hydrophobic mono- or poly-vinyl monomers or mixtures thereof and the monovinyl monomers are selected from the group consisting of alkyl acrylates, alkyl methacrylates, aryl acrylates, aryl methacrylates, aryl alkyl acrylates, aryl alkyl methacrylates, fluorinated alkyl acrylates, fully or partially fluorinated alkyl methacrylates, for example 2,2,3,3,3-pentafluoropropyl methacrylate, styrene, vinylnaphthalene or vinylanthracene and derivatives thereof, wherein the alkyl group in each of the alkyl monomers has 1-20 carbon atoms.

In a more preferred embodiment of the method for the manufacture of the patterned substrate according to the present invention, the hydrophobic pattern is produced by photografting of the hydrophilic porous polymer layer with hydrophobic mono- or poly-vinyl monomers or mixtures thereof and the polyvinyl monomers are selected from the group consisting of alkylene diacrylates, alkylene dimethacrylates, pentaerythritol tetraacrylates, pentaerythritol tetramethacrylates, trimethylopropane acrylates, trimethylopropane methacrylates, divinylbenzenes and divinylnaphthalenes.

In a preferred embodiment of the present invention, the modification of the surface of the porous layer in step (d) of the method for the manufacture of a patterned substrate according to the present invention is carried out by photografting the surface of the porous polymer layer using a photomask, wherein a photografting mixture is provided on the surface of the porous polymer layer. Said photografting creates a pattern of hydrophobic, preferably superhydrophobic, barriers separating hydrophilic, preferably superhydrophilic, areas. As described earlier, the photomask may be applied so as to create the pattern, or to create the areas.

According to the present invention, the polymerization of the polymerizing mixture affords a hydrophilic, preferably superhydrophilic, polymer layer which is subsequently photografted with a compound capable to impart superhydrophobicity to the porous polymer layer using a photomask. Thus, the hydrophilic, preferably superhydrophilic, areas originate from the regions of the porous polymer layer which have not been photografted. In addition, the regions which are photografted are transformed into hydrophobic, preferably superhydrophobic, barriers separating the hydrophilic, preferably superhydrophilic, areas.

According to the present invention, the porous polymer layer is a HEMAcoEDMA polymer layer, and the hydrophobic, preferably superhydrophobic, barriers are created by photografting the HEMAcoEDMA surface with a photografting mixture. Particularly preferred is a photografting mixture containing 2-,2-,3-,3-,3-pentafluoropropyl methacrylate (PFPMA). In a more preferred embodiment of the present invention, the hydrophilic porous polymer surface comprises poly(2-hydroxyethyl methacrylate-co-ethylene dimethacrylate) and the photografted hydrophobic monomer is 2,2,3,3,3-pentafluoropropyl methacrylate.

According to a further preferred embodiment of the present invention, the photografting mixture consists of 15wt.% of 2,2,3,3,3-pentafluoropropyl methacrylate and 1wt.% of ethylene dimethacrylate in 1/3 (v./v.) mixture of water - tert-butanol containing 0.25wt.% benzophenon.

In a preferred embodiment of the method for the manufacture of the patterned substrate according to the present invention, the hydrophilic porous polymer surface comprises HEMAcoEDMA and the photografted hydrophobic monomer is PFPMA. In a particularly preferred embodiment of the method for the manufacture of the patterned substrate according to the present invention, a 12.5 um-thin hydrophilic porous HEMAcoEDMA layer is patterned with PFPMA to form an array of square hydrophilic HEMAcoEDMA areas separated by hydrophobic areas.

The preparation of a patterned substrate ready for further applications such as printing biomolecules takes preferably about one hour.

The method for the manufacture of a patterned substrate according to the present invention preferably further comprises the step (e) providing at least one biologically active agent in at least one hydrophilic, preferably superhydrophilic, area, more preferably in each of the hydrophilic, preferably superhydrophilic, areas.

In another preferred embodiment of the present invention, the method for the manufacture of the patterned substrate further comprises step (f) seeding cells to be cultivated into at least one hydrophilic, preferably superhydrophilic, area, more preferably into each of the hydrophilic, preferably superhydrophilic, areas or onto the whole area of the substrate coated with the photografted porous polymer layer. In a preferred embodiment of the present invention, the method for the manufacture of the patterned substrate according to the present invention, comprises a step (f) providing at least one type of cells in at least one hydrophilic area of the pattern. In another preferred embodiment of the present invention, the method for the manufacture of the patterned substrate according to the present invention, comprises a step (f) providing at least one type of cells on the whole area of the patterned substrate. In a further preferred embodiment of the present invention, the method for the manufacture of the patterned substrate according to the present invention, comprises a step (f) seeding cells to be cultivated into at least one superhydrophilic area.

The present invention further provides a method for creating predefined patterns of cells by cultivating them on a patterned substrate as defined herein, wherein the geometry of the cell pattern is determined by the geometry of the hydrophilic, preferably superhydrophilic,, areas surrounded by hydrophobic, preferably superhydrophobic,, areas. In a preferred embodiment, the pattern formed by cells is a cell array, wherein the cells are in addressable positions which are predefined by the patterned substrate. In a preferred embodiment of the present invention, the pattern formed by the cells is obtained by providing a patterned substrate as defined herein having at least one biologically active agent as defined herein in at least one hydrophilic, preferably superhydrophilic, area. In a particularly preferred embodiment, when cells are seeded on the patterned substrate, the at least one biologically active agent binds to molecules present on the surface of at least on cell or interacts with patterned cells in a different way and thus, provides the bound cell with a detectable signal as defined herein. By detecting the detectable substance, the position of the bound cell is known and is addressable. In another preferred embodiment of the present invention, the pattern formed by the cells is obtained by selectively seeding cells into at least one predetermined hydrophilic, preferably superhydrophilic, area of the patterned substrate as defined herein. By seeding cells into predetermined hydrophilic areas the position of the cells is addressable.

The present invention further provides a method for cultivating cells comprising the steps of:
(I) providing a patterned substrate according to the present invention;
(II) seeding cells to be cultivated into at least one hydrophilic area of the patterned substrate; and
(III) cultivating the seeded cells.

Within the scope of the invention, the term "cells" means a generic term and encompass the cultivation of individual cells, tissues, organs, insect cells, avian cells, mammalian or bacterial cells, primary cells, continuous cell lines, stem cells and/or genetically engineered cells, such as recombinant cells expressing a hetereologous polypeptide or protein. Recombinant cells include, for example, cells expressing heterologous polypeptides or proteins, such as a growth factor or a blood factor.

Mammalian cells suitable for cultivation in the method of the present invention include those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells that can be cultivated in the method according to the present invention can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, HeLa cells, NS-1 cells MRC-5 cells, WI-38 cells, BHK cells, 293 cells, mouse mammary carcinoma cells, rat mammary carcinoma cells, HEK cells, HEPA cells, and RK-cells. In a preferred embodiment of the present application, the cells cultivated in the method according to the present invention are selected from the group, consisting of mouse mammary carcinoma cells, rat mammary carcinoma cells, HEK cells, HEPA cells, and HeLa cells.

The cells can be cultivated in any suitable medium known in the art under any suitable conditions, e.g. CO₂ content, temperature, or pH known in the art.

The medium used for cultivation the cells in the method of the present invention may be a synthetic medium, such as DMEM, HAM's F12, Medium 199 or RPMI, or combinations thereof, and others that are known from the literature or are commercially available, which may be supplemented with animal proteins, for example by supplementing it with fetal calf serum or bovine serum. The basal medium can comprise a number of ingredients, including amino acids, vitamins, organic and inorganic salts, sources of carbohydrate, each ingredient being present in an amount which supports the cultivation of a cell *in vitro.* For example, DMEM/HAM's F12 (1:1) medium as basal medium can be used. The medium may contain auxiliary substances, such as buffer substances like sodium bicarbonate, oxidation stabilizers, stabilizers to counteract mechanical stress, or protease inhibitors.

The term "cultivation," in its various grammatical forms, refers to the maintenance of the cells *in vitro* under conditions permissive for growth and continued viability. Mammalian cells are typically cultivated in a cell incubator at about 37°C, with the culture medium having an optimal pH in the range of about 6.8 to 7.6, preferably between 7.0 and 7.3. Cells might have a complete medium change about every 2 to 3 days, or more or less frequently, if required. Cultivation approaches can include, depending on context and need, the sub-cultivation, passaging and propagation of the cells.

According to the present invention, the patterned substrate provided in the method for cultivating cells according to the present invention is a patterned substrate as defined herein.

In a preferred embodiment of the present invention, at least one hydrophilic, preferably superhydrophilic, area of the patterned substrate provided in step (I) of the method for cultivating cells as defined herein contain at least one biologically active agent. In a preferred embodiment of the present application the biologically active agent is as defined herein.

In a more preferred embodiment of the present application, a biologically active agent is a nucleic acid molecule, more preferably a nucleic acid molecule being selected from the group consisting of DNA, RNA, and iRNA agents. In a particularly preferred embodiment of the present application, the biologically active agent is a nucleic acid and at least one cell as defined herein is transfected with the nucleic acid after the seeding step (II) and before the cultivating step (III), i.e. prior to cultivation in the hydrophilic, preferably superhydrophilic, area. In a more preferred embodiment, the nucleic acid encodes a gene product which provides the cell being transfected with the nucleic acid with a detectable feature upon expression by the cell. In another preferred embodiment of the present invention, the nucleic acid is an iRNA agent and the expression of the gene product targeted by the iRNA agent is significantly decreased after transfection of the cell with the iRNA agent.

In yet another preferred embodiment of the present invention, the method for cultivating cells further comprises a step of (IV) detecting cells transfected with at least one iRNA agent. In one embodiment of the present invention, the cultivated cells are detected before the seeding step (II), before the cultivating step (III), during the cultivating step (III) and/or after the cultivating step (III) of the method for cultivating cells as defined herein. The detection can be carried out for example by detecting a peptide and/or nucleic acid indicating the cell. The peptide and/or nucleic acid may be expressed by the cell. In a preferred embodiment of the present invention, the peptide and/or nucleic acid using a compound binding to the peptide and/or nucleic acid having a detectable label. In one embodiment of the present invention, the compound having a detectable label is a nucleic acid biologically active agents binding specifically to a nucleic acid isolated from a cultivated cell. In another preferred embodiment of the present invention, the compound having a detectable label is an antibody or antibody fragment binding specifically to a peptide isolated from or presented on the surface of a cultivated cell.

In one embodiment of the present application, the detection of the cultivated cell is carried out before the cultivating step (III) and the detection enables the counting of the cells. Using the information obtained by counting the cells, a predetermined number of cells is seeded into the hydrophilic, preferably superhydrophilic, areas.

The term "detectable label" does not exhibit any particular limitation and may be selected from the group consisting of radioactive labels, fluorescent dyes, compounds having an enzymatic activity, magnetic labels, antigens, and compounds having a high binding affinity for a detectable label. For example, a radioactively or fluorescently labeled compound can be used to detect nucleic acids or peptides isolated from or being presented on the cultivated cells, e.g. after electrophoresis. Fluorescent dyes linked to a probe may serve as a detection label for nucleic acids, e.g. in a real-time PCR. A substance having an enzymatic reactivity such as the enzyme luciferase which produces a light signal upon contact with the respective substrate can also be used as a detectable label which may be linked covalently to said compound having a detectable label. Labeling the compound of the present invention with magnetic beads allows a selective extraction of nucleic acids or peptides isolated from or being presented on the cultivated cells. Coupling a compound having a detectable label to an antigen allows the detection of the compound by an antibody/enzyme-complex (the enzyme being e.g. phosphatase) catalyzing a detectable color reaction when using a suitable substrate. A substance with a high binding affinity for a different detectable label such as biotin which binds to a detectable label covalently linked to e.g. streptavidine, is a further possibility for making a compound of the present invention detectable.

The detection step (IV) of the above-defined method may comprise one or more detection method(s) selected from the group consisting of immunoblotting, immunoprecipitation, immunocapture, monoclonal antibody immobilization of platelet antigens or enzyme linked immuno sorbent assay (ELISA), flow cytometry, protein array technology, spectroscopy, mass spectrometry, chromatography, surface plasmonic resonance, fluorescence extinction and/or fluorescence energy transfer. The detection method can, for example, be selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of the person skilled in the art to choose the optimal parameters, such as buffer system, temperature and pH for the respective detection system to be used.

In a preferred embodiment of the present invention, the method for cultivating cells as defined herein comprises the step of bringing the cell into contact with a biologically active agent, preferably a pharmaceutically effective substance during or before the seeding step (II) and/or during the cultivating step (III) and detecting the influence of a treatment with a biologically active agent on the cells cultivated in the cultivating step (III). The biologically active agent compound according to the present invention can be any biologically active agent known in the art. In one preferred embodiment of the present invention, the biologically active agent is deposited in hydrophilic, preferably superhydrophilic, areas of the cell array of the present invention before the seeding step (II) and the cells are brought into contact with the biologically active agent during the seeding step (II). In another preferred embodiment of the present invention the cells are brought into contact with the biologically active agent during the cultivating step (III). The influence of a treatment with a biologically active agent on the cells can be detected by detecting alterations of the cell culture, preferably alterations in the morphology of cells, behavior of cells, such as motility, division, proliferation, adhesion, differentiation, expression of peptides and/or nucleic acids using the detection methods defined above. In a preferred embodiment of the alterations of the cell culture are detected using microscopy.

In a particularly preferred embodiment of the present invention, the influence of a treatment with the biologically active agent on the cells is detected by measuring differences in the gene expression profile of the cells. The term "gene expression profile" as used herein refers to the relative expression of a plurality of mRNA transcripts or post-transcriptional level including protein amounts and post-translational modifications. A gene expression profile of a cell reflects the amount of each mRNA transcript and/or post-transcriptional level in the starting sample. Methods for analyzing the gene expression profile of a cell are known in the art. In a preferred embodiment of the present invention the gene expression profiles of the cells is obtained using e.g. microarray techniques, PCR, microscopy or Northern-analysis. In a particularly preferred embodiment of the present invention, the biologically active agent is an iRNA agent.

In a preferred embodiment of the method for cultivating cells according to the present invention, the seeding of cells to be cultivated into at least one hydrophilic area of the patterned substrate in step (II) is a seeding of cells to be cultivated onto the whole patterned substrate.

In another preferred embodiment of the method for cultivating cells according to the present invention, the at least one biologically active agent is a nucleic acid molecule, preferably selected from the group consisting of iRNA, plasmids, DNA, and the method further comprises the step of (IV) detecting cells transfected with at least one nucleic acid molecule.

In a preferred embodiment of the present invention, the method for cultivating cells according to the present invention is used for patterning standardized high-density cell arrays. Those can be used for routine combinatorial high-throughput (CHT) iRNA-screens or for any other cell-screening experiments that involve large biomolecule-libraries.

The transfection of cell clusters on an array can be for example reverse transfection. In a particularly preferred embodiment of the present invention, the patterned substrate is preprinted with transfection reagents before the seeding step of the method for cultivating cells according to the present invention.

According to the present invention, the seeding step (II) of the method of cultivating cells as defined herein may be equal to the seeding step (f) of the method for the manufacture of the patterned substrate as defined herein.

Described herein is a method for cultivating cells which comprises the steps of:
(a) providing a polymerization mixture between two solid supports,
(b) polymerizing the polymerization mixture to form a porous polymer layer,
(c) removing one of the two solid supports,
(d) photografting the porous polymer layer using a photomask, thereby creating a hydrophobic, preferably superhydrophobic, pattern of fluorinated hydrophobic, preferably superhydrophobic, barriers separating hydrophilic, preferably superhydrophilic, areas to obtain a patterned substrate,
(e) providing at least one biologically active agent in at least one hydrophilic, preferably superhydrophilic, area,
(f) seeding cells to be cultivated into at least one hydrophilic, preferably superhydrophilic, area of the patterned substrate, and
(g) cultivating the seeded cells,
which may optionally further comprise the step (h) detecting cells transfected with at least one iRNA agent which may be equal to the aforementioned detecting step (IV) of the method for cultivating cells.

Additionally, the present invention provides a use of a patterned substrate according to the present invention for cultivating mammalian or prokaryotic cells.

In a preferred embodiment of the present invention, the use of a patterned substrate further comprises (iii) at least one biologically active agent provided in at least one hydrophilic, preferably superhydrophilic, area of the patterned substrate.

According to the present invention, the patterned substrate is a patterned substrate as defined herein. In a preferred embodiment of the present invention, the cells and/or the cultivation of cells are as defined herein.

In a further preferred embodiment of the use of a patterned substrate according to the present invention, the cultivated mammalian or prokaryotic cells form patterns of eukaryotic or prokaryotic cells.

The present invention relates to a use of a patterned substrate as defined herein for different cell experiments, preferably selected from the group, consisting of RNAi screens, protein arrays, drug screens, expression of recombinant proteins, screenings of interactions of cells with polymer or hydrogel samples.

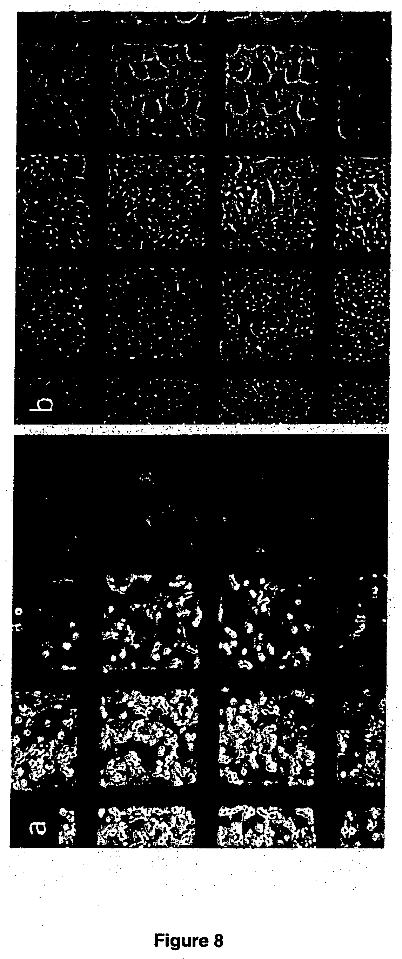

According to the present invention, a method for the preparation of a patterned substrate has been developed with sharp defined hydrophilic areas separated by narrow hydrophobic, preferably superhydrophobic, barriers. Within the scope of the present invention, it could be showed that hydrophilic areas can be used as micro-reservoirs for water-based solutions separated by watertight barriers (Fig. 3c), while hydrophobic, preferably superhydrophobic, areas served as extremely efficient barriers for cell migration (Fig. 8).

The porous hydrophilic, preferably superhydrophilic, polymer layer, poly(2-hydroxyethyl methacrylate-co-ethylene dimethacrylate (HEMAcoEDMA), proved itself as a suitable material for culturing of cells, for example HeLa (the most common cell line in biological laboratories) and fluorescent rat mammary carcinoma cells. The surface of the porous polymer layer can be patterned with hydrophobic functionality and hydrophilic functionality, respectively, using for example UV-initiated photografting. Cells preference for wetted, rough surfaces has recently been shown.[9]

This also seems to be evident for the superwetting and easily protein adsorbing hydrophilic, preferably superhydrophilic, areas of the patterned substrate according to the present invention. The excellent cell adhesion to the rough surfaces in combination with the ability to easily pattern highly cell-resistant hydrophobic, preferably superhydrophobic, regions and/or barriers in the same material represents a completely novel and powerful approach for controlling cell behavior. Moreover, no biomolecule-degenerating effect is to be expected because of the solvent-like physical adsorption in the hydrated porous structure of the hydrophilic, preferably superhydrophilic, areas.

The patterned substrate according to the present invention is perfectly suitable for iRNA-based high throughput screens of gene-functions and other high-throughput-screening experiments. Those screens usually need one or two days from seeding until analysis. With the right cell concentration in the seeding-suspension, no cell-bridges to adjacent areas and no cell migration is to be expected in this short time span. If contact-inhibited cells are used, bridge formation should not be expected even for longer time spans. The hydrophobic, preferably superhydrophobic, barriers have no topographical barrier and therefore do not hamper microscopy. On the other hand, they are easily distinguishable from the hydrophilic, preferably superhydrophilic, areas under microscope. This allows fast orientation for automated machinery during the running experiment. Compared to other cell arrays, this new format is not only much denser and magnitudes less expensive - it is also more robust than others and faster to produce. For the first time, the area to area distance could be scaled down as far as it is needed for making a functional single cell array for genome-wide RNAi cell screens. The technique presented here allows pushing the amount of areas on a single plate towards its theoretical maximum, which is given by the minimum area needed per area. The preparation of the here developed patterned substrate needs about one hour. All used materials are inexpensive.

The decreased amount of iRNA needed for each array and the possibility to store a pre-printed array up to 15 month[2] might make this technique a routine method for RNAi-screening affordable to any biological laboratory.

Finally, the here presented facile but highly effective method to pattern cells has a great potential not only for microarrays but also in other research areas that require cells in a predefined spatial order. Further, the hydrophilic/hydrophobic patterns of the patterned substance according to the present invention can be used for making advanced standard microplates and/or microfluidic devices, like lateralflow devices, or diagnostic devices.

In order to create a patterned substrate with a area density beyond the today used arrays several problems have to be overcome:
(1) Cross contamination has to be prevented. An array made from probe molecules simply printed on a glass substrate cannot be very dense, because at some point the spotting solutions of adjacent areas will mix.
(2) Cell migration between adjacent areas has to be eliminated or at least kept to a minimum.
(3) The array has to allow investigation with common screening techniques. It should be transparent and possess a flat surface.
(4) It should be highly homogeneous and all features (geometry, volume, size of areas) should be strictly controllable to guarantee high data-quality.
(5) It should be biocompatible, mechanically stable and not degrade within a reasonable time-span.

Only the simultaneous solution to all those problems will allow a significant progress in the development of cell arrays. Today, solutions that only address some of those problems exist. The array developed by Reyman et. al. solved the problem of cross-contamination, what allowed them to increase the density by a factor of six, but the lack of a possibility to control cell-migration and area-geometry prevented further development. Thus, even in this highly advanced array the unused space greatly exceeds the space actually used for transfection experiments. With increasing area-density cell-migration gains more and more influence and the systematical error would quickly become too big for obtaining valid data. For dense packing not only the migration has to be prevented, but also surface chemistry has to be controlled to such a degree, that the spotting-solution automatically spreads in square-shaped areas with very low area-to-area-distances during the printing process. This has to be done on a substrate with good optical properties (transparent, flat) and without degenerating interactions with the probe molecules. The transparent TiO₂-array mentioned before can be patterned in an efficient way, but a method to prevent cell-migration is lacking and it could also cause biocompatibility-problems if it is not kept in the dark, not to mention the light sensitivity of the material itself. It also probably lacks sufficient mechanical stability.

All recent approaches on cell arrays are quite complicated in their design and production, what makes them expensive. Only a low price would make those arrays accessible for broad fields of biological research.

Using a polymer like the before mentioned HEMAcoEDMA, porosity and free volume can be independently controlled via the porogen-composition and the fraction of porogen in the polymerization-mixture. The monolithic character of those polymers also gives them remarkably mechanical strength.

Compared to other cell array-formats the patterned substrate according to the present invention has several advantages. It is the first technique that allows controlling cell migration in a way that is applicable to transfection-arrays. The possibility to pattern hydrophobic, preferably superhydrophobic, structures of any geometry and size allows to create a high-density array of hydrophilic, preferably superhydrophilic, areas separated by narrow hydrophobic, preferably superhydrophobic, barriers. The extreme water repellency of the hydrophobic, preferably superhydrophobic, barriers prevents any cross-contamination between adjacent areas. The material is preferably transparent and flat. Besides, the production cost of the patterned substrate is extremely low as well as the production time.

Printed with probe-molecules, the patterned substrate can be seeded with cells and the effect of the probe molecules on them can be investigated in a high-throughput application.

The figures show:
Figure 1: Production of a patterned substrate according to one embodiment of the present invention to obtain a porous polymer layer with a defined thickness, the polymerization is carried out in a mold between two glass substrates (glassplates). The layer is subsequently photografted with a photomask.
Figure 2: (a) Edge of a patterned substrate. (b) Dimensions of the patterned substrate.
Figure 3: (a) XPS measurements of the fluorine occupation on a 8 x 4 mm² area. Surfaces of: (a): black line. (b): blue line. (c): red line. (b) Wetted with water the nanoporous HEMAcoEDMA (Mixture 1) turns nearly completely transparent while it significantly scatters light in the dry state. (c) Each spot of the array is adressable on its own and can for example be colored with water based dyes with a small needle while the surrounding superhydrophobic barriers remain dry and colorless.
Figure 4: Contact angle measurements of: (a) HEMAcoEDMA copolymer. (b) PFPMA photografted HEMAcoEDMA copolymer.
Figure 5: Scanning electron microscopy images. Surface of the nanoporous poly(HEMAcoEDMA) with a photografted micropattern (a,b). Surface of the microporous poly(HEMAcoEDMA) (c).
Figure 6: Fluorescent microscope image of DAPI-stained HeLa cells (3^{rd} day after seeding) on a nanoporous HEMAcoEDMA-plate (Mixture 1) photopatterned with PFPMA-squares. Scale bar: 500 µm.
Figure 7: Fluorescent rat mammary carcinoma cells (CMV-mCherry neo marked) seeded on the array. (a) 20 minutes after seeding - cells precipitated everywhere. (b) After one day. (c) After two days. (d) Cells seeded on the array with barriers in the Wenzel-state, two days after seeding.
Figure 8: (a) HeLa Cells seeded in a drop of medium for one day, than the whole array was kept in medium for two days (b). No migration occurred.

The present invention will now be further illustrated in the following examples without being limited thereto.

### Example

### 1) Material and Methods:

### Experimental Details

Materials and Instrumentation: All polymerizations and photograftings were carried out on an OAI Model 30 deep UV collimated light source (San Jose, CA, USA) fitted with a USHIO 500 W HgXE lamp (Japan). Irradiation power was calibrated to 12.0 mW/cm² (5.10 mW/cm² after cover plate, 4.77 mW/cm² after cover plate and photomask) using an OAI 360 UV Powermeter with a 260 nm probe head. Scanning electron microscopy images were obtained using the LEO 1530 SEM. The samples were gold sputtered with 15 nm gold using the Balzers Union MED 10. For UV-VIS-NIS spectra, a Micropack DH-2000-BAL UV-Vis-NIR-Lightsource was used. XPS measurements were carried out on a Leybold-Heraeus MAX200 with an EA200 hemispheric energy-analysator as energy dispersive element and a 300 W magnesium anode as x-ray source. ImageJ software with a DropSnake plugin was used to measure static and dynamic contact angles. Schott Nexterion Glass B UV transparent glass plates were used as substrates for smaller polymer layers and arrays as well as for full-scaled arrays.

Butyl methacrylate was purchased from Fluka, all other monomers from Sigma-Aldrich. Monomers were purified before usage by passing them through a small column packed with aluminum oxide to remove the inhibitors. Biochemicals were purchased from Invitrogen. Fig. 3c was obtained via addressing alternating spots with a cutoff 0.3 mm needle. Blue spots were colored with methylene blue (8 g × L-1), red spots with neutral red (8 g × L-1). Cell lines were provided by the Institute of Toxicology and Genetics (Karlsruhe Institute of Technology).

### Polymerization mixtures:

- (Mixture 1) Nanoporous, transparent poly(2-hydroxyethyl methacrylate-co-ethylene dimethacrylate) (HEMAcoEDMA): 2-hydroxyethyl methacrylate (HEMA) (24wt.%), ethylene dimethacrylate (EDMA) (16wt.%), 1-decanol (12wt.%), Cyclohexanol (48wt.%) and 2,2-dimethoxy-2-phenylacetophenone (DMPAP) (1wt.% with respect to monomers).
- (Mixture 2) Microporous, non transparent poly(2-hydroxyethyl methacrylate-co-ethylene dimethacrylate): 2-hydroxyethyl methacrylate (24wt.%), ethylene dimethacrylate (16wt.%), 1-decanol (40wt.%), cyclohexanol (20wt.%) and 2,2-dimethoxy-2-phenylacetophenone (1wt.% with respect to monomers).
- (Mixture 3) Microporous, non transparent poly(butyl methacrylate-co-ethylene dimethacrylate) (BMAcoEDMA): butyl methacrylate (BMA) (24wt.%), ethylene dimethacrylate (16wt.%), 1-decanol (40wt.%), cyclohexanol (20wt.%) and 2,2-dimethoxy-2-phenylacetophenone (1wt.% with respect to monomers).

### Photografting mixture:

- (Photomixture) Crosslinked 2,2,3,3,3-pentafluoropropyl methacrylate grafting-mixture: 15wt.% 2,2,3,3,3-pentafluoropropyl methacrylate, 1wt.% ethylene dimethacrylate in 1/3 (v./v.) mixture of water - tert-butanol containing 0.25wt.% benzophenon

### Glass Surface Modification

To achieve covalent attachment of the polymer layer, the glass surfaces were first activated, than functionalized with an anchor-group for methacrylates. Cleaned glass plates were immersed in 1 M NaOH for one hour and afterwards washed with deionized water followed by immersing them in 1 M HCl for 30 min, washing with deionized water and drying with a nitrogen gun.

Modification of glass plates: Several drops of a solution containing 20% 3-(trimethoxysilyl)propyl methacrylate in Ethanol adjusted to pH = 5 with acetic acid were dropped on an activated glass plate. The plate was covered with another activated glass plate. It was made sure that no bubbles were trapped between the two plates. The solution was reapplied after 30 minutes and after another 30 minutes the plates were washed with acetone and dried with a nitrogen-gun.

Fluorination of glass plates: An activated glass plate was placed in a vacuumated dessicator together with a vial containing tridecafluor-(1,1,2,2)-tetrahydrooctyltrichlorosilane for 2h.

### Polymerization Procedure and Photografting

Two stripes thin Teflon film (American Durafilm Co.), defining the thickness of the polymer layer, were placed at the edges of a glass plate and another glass plate was stapled on top of it. The polymerization mixture was injected in the mold between them and irradiated for 15 min with 12.0 mW/cm² 260 nm UV-light. The mold was then carefully opened using a scalpel.

Microporous polymers: Two plates modified with the methacrylate-anchor were used for polymerization. After polymerization, the plates were separated using a scalpel. A thick layer adhered to the top-plate while a very thin layer adhered to the bottom plate. The plates were extensively washed with methanol or ethanol and kept in methanol for some minutes.

Nanoporous polymers: As the nanoporous polymer layers possessed much higher mechanical strength than the microporous layers, separating two anchor-group-modified glass plates with the layer between them resulted in very inhomogeneous surfaces where differing parts of the layer adhere to only one plate. To achieve better separation, a fluorinated plate was used as bottom plate. After the polymerization-process finished they could easily be separated and the whole layer adhered to the silanized top-plate.

Photografting: The polymer layer was wetted with the respective photografting mixture and covered with a fluorinated plate. The photomask was placed on top and it was irradiated for 30 min. The obtained pattern was washed extensively with methanol or ethanol and kept in methanol for some hours.

### Cell Studies

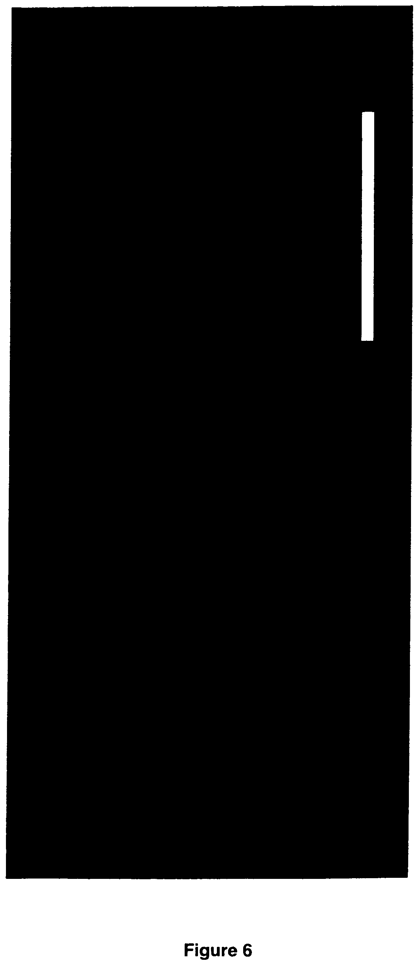

All cell-culturing took place under the clean bench. Cell suspension was obtained via trypsinizing a culture that grew in a Petri dish for 2-3 days. Trypsination was stopped by adding 10 ml Dulbecco's Modified Eagle's Medium (DMEM). For sterilization the polymer-coated glass-plates were kept in ethanol for some minutes, dried on air and placed in Petri dishes. They were covered with medium (10% of fetal bovine serum (FBS) in DMEM) so that the plates were covered completely. Then, cell-suspension was added. The dishes were incubated at 37 °C. 4',6-diamidino-2-phenylindole (DAPI) or DRAQ5 were used for staining HeLa cells (1/10000 v./v.). For the migration experiments of Fig. 6 cells were cultured for 2 days in a 0.05 ml drop (230000 cells × ml-1) on the micropattern, than the whole array was immersed in 15 ml medium within a 10 cm Petri dish. For the experiments of Fig. 7 fluorescent rat mammary carcinoma cells were seeded on an array with a density of 17500 cells × cm-2. Fig. 7d was obtained via seeding the cells on an array completely in Wenzel state. The array was sterilized and wetted with ethanol, than extensively washed with sterile phosphate buffered saline (PBS) to exchange all ethanol before seeding it.

### 2) Results:

### Characterization of the Materials

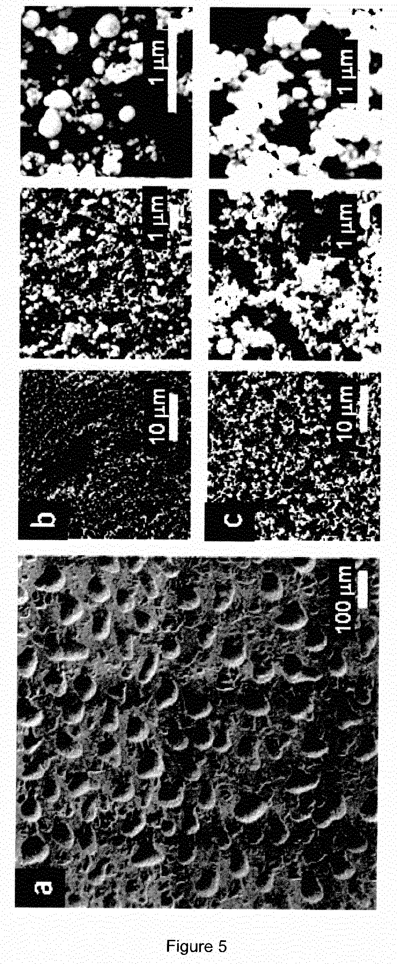

The developed materials were characterized in their wetting behavior, surface-composition and topography. A microporous HEMAcoEDMA (Mix. 2) has a static water contact angle (WCA) θₛₜₐₜ of only 14°. As microporous polymers with typical poresizes of about 1-2 µm (Fig. 5c) do not possess sufficient transparency, the typical pore size had to be decreased below the wavelength of visible light. This could be achieved via increasing the amount of cyclohexanol from initial 20 %wt (Mix. 2) to 48 %wt (Mix. 1). The decrease in poresize to about 150-250 nm (Fig. 5b) resulted only in a slight increase in θₛₜₐₜ up to 15° (Fig. 2a). To further tune transparency, different layer thicknesses were investigated. UV-driven photografting of an array with watertight spots and good transparency works with layers in the micrometer-range, for our prototype we chose a thickness of 12.5 µm. With this thickness and being wetted with water, the transparency of the respective nanoporous HEMAcoEDMA increased to the point of complete transparency (Fig. 3b)

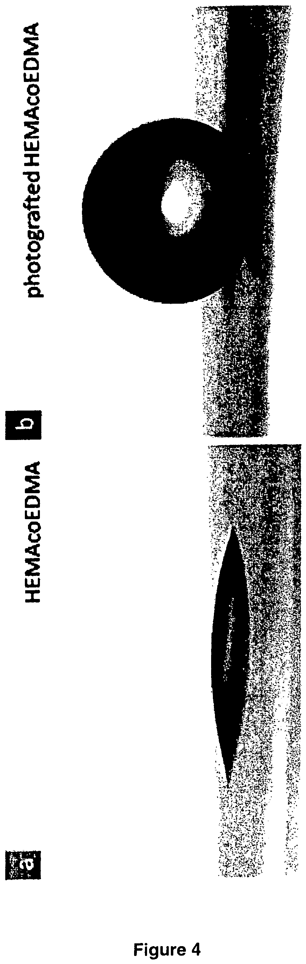

The pattern was investigated with X-ray photoelectron spectroscopy (XPS) and compared to a non photografted HEMAcoEDMA and a completely photografted HEMAcoEDMA (Fig. 2d). This made evident that occupation of fluorine on the surface of small photografted features is drastically higher than on large photografted zones - a consequence of the photografting-process itself. This effect renders the 60 µm thin barriers extremely hydrophobic, but does not allow the creation of a large surface with the same composition as a small photografted zone. Nevertheless a nanoporous HEMAcoEDMA photografted with PFPMA/EDMA on a large area possesses static θₛₜₐₜ (Fig. 4b), advancing θ_{adv} and receding θ_{rec} WCAs of 165°, 167° and 157° respectively. A microporous poly(butyl methacrylate-co-ethylene dimethacrylate) (BMAcoEDMA) (Mix. 3), which is well known for its superhydrophobic properties,[1] shows static θₛₜₐₜ, advancing θ_{adv} and receding θ_{rec} water contact angles of only 153°, 161°, 151°. Despite the loss of microporosity the contact angles of the PFPMA-photografted HEMAcoEDMA are even higher than for microporous BMAcoEDMA, clearly an effect of the hydrophobicity of the fluorinated side chains.

XPS-measurements showed that the photografting resulted in a chemical modification of the surface (Fig. 3a) while scanning electron microscopy (SEM) confirmed that the topographical modification of the photografted area was insignificant (Fig. 5a). Figures 5b, c show the morphologies of the micro- and nanoporous polymer films.

### Cell Studies

Cell studies were carried out with HeLa cells and fluorescent rat mammary carcinoma cells (CMV-mCherry neo, Tsien Lab). In a first experiment HeLa cells were seeded on a nanoporous HEMAcoEDMA-layer with photogafted PFPMA-features on it. The HEMAcoEDMA-spots were immediately wetted in the growth medium, while the photografted superhydrophobic zones remained dry for several days. After staining the cells with 4',6-diamidin-2-phenylindol (DAPI) almost no cells could be seen in the superhydrophobic zones (Fig. 6). However, no difference in cell occupation could be noticed when using microporous HEMAcoEDMA-plates with hydrophobic features or microporous poly(butyl methacrylate-co-ethylene dimethacrylate) (BMAcoEDMA) with hydrophilic features.

The cell array developed for the transfection experiments was also studied with regard to the behavior of fluorescent rat mammary carcinoma cells. Cells were homogeneously distributed across the array several minutes after seeding (Fig. 7a). The cells started adhering to the protein adsorbing HEMAcoEDMA spots immediately after precipitating. After one day, the cell-occupation on the hydrophilic spots greatly outclassed the occupation of the superhydrophobic zones (Fig. 7b). After two days, the hydrophilic spots were occupied completely, while the superhydrophobic barriers contained almost the same number of cells as immediately after seeding (Fig. 7c). According to the microscopic images, the ratio of cell density on the hydrophilic regions to that on the hydrophobic regions is approximately 1 for figure 7a, 3.5 for 7b, 6 for 7c and 3.5 for 7d. As the used cancer-cells were not cell-contact inhibited in their growing behavior, the high occupation of the surface allowed them to form bridges that ultimately led to spreading into adjacent spots. This seemed to be the only possibility for the cells to cross the superhydrophobic zones (Fig. 7c).

It is well known that surfaces that cannot adsorb proteins also show efficient cell resistant behavior. This is a result of the fact that cells preferably adhere to the surfaces coated with protein layers similar to those of their extracellular matrix. Moderate protein repellant and antifouling properties of fluorinated surfaces were reported.[6] In our case the protein repellant property of the photografted areas is combined with the superhydrophobic property meaning that only a small fraction of the solid is in contact with the medium. Thus, this combination leads to the observed surprisingly strong cell-resistivity of the superhydrophobic barriers with the width of only three cell diameters.

However, this hypothesis implies that this combination should only exist as long as the superhydrophobic surface is in the Cassie-Baxter mode. Its transition into the Wenzel-state would reduce the cell-resistivity because of the increased solid area being in contact with the medium.

To test this hypothesis the cell growth experiment was performed on the surface of nanoporous HEMAcoEDMA with photografted PFPMA pattern that was artificially transferred into the Wenzel state before the experiment (Fig. 7d). Artificial transition to the Wenzel state was achieved via wetting the array with ethanol. The solvent was than exchanged with PBS-buffer followed by cell seeding. The fact that the whole array was in the Wenzel state became manifest from the microscope images of the array in the bright field mode that showed complete transparency of the PFPMA-patterned areas. The result of this cell experiment showed the decrease of the cell-resistant efficiency of the photografted areas after they were transferred into the Wenzel state (compare Fig. 7c and 7d).

To prove that the barriers prevent cell-migration, HeLa-cells were grown for two days in a droplet of cell medium placed on the array surface and surrounded by the dry area. After two days, the whole array was wetted with the medium and it was incubated further. Four days after, the array was imaged and no cells were found in the area around the initial position of the droplet, i.e. no migration across the barriers could be observed (Fig. 8a,b).

### Reference List

[1.] P. A. Levkin, F. Svec, J. M. J. Frechet, Advanced Functional Materials 2009, 19, 1993-1998.
[2.] H. Erfle, B. Neumann, U. Liebel, P. Rogers, M. Held, T. Walter, J. Ellenberg, R. Pepperkok, Nature Protocols 2007, 2, 392-399.
[3.] J. Ziauddin, D. M. Sabatini, Nature 2001, 411, 107-110.
[4.] B. Neumann, T. Walter, J. K. Heriche, J. Bulkescher, H. Erfle, C. Conrad, P. Rogers, I. Poser, M. Held, U. Liebel, C. Cetin, F. Sieckmann, G. Pau, R. Kabbe, A. Wunsche, V. Satagopam, M. H. A. Schmitz, C. Chapuis, D. W. Gerlich, R. Schneider, R. Eils, W. Huber, J. M. Peters, A. A. Hyman, R. Durbin, R. Pepperkok, J. Ellenberg, Nature 2010, 464, 721-727.
[5.] H. Erfle, B. Neumann, P. Rogers, J. Bulkescher, J. Ellenberg, R. Pepperkok, Journal of Biomolecular Screening 2008, 13, 575-580.
[6.] N. Vandencasteele, B. Nisol, P. Viville, R. Lazzaroni, D. G. Castner, F. Reniers, Plasma Process. Polym. 2008, 5, 661-671.
[7.] X. Zhang, H. K, Z. Liu, S. Nishimoto, D. A. Tryk, T. Murakami, H. Sakai, M. Abe, A. Fujishima, Chemical Communications 2007, 46, 4949-4951.
[8.] J. Reymann, N. Beil, J. Beneke, P. Kaletta, K. Burkert, H. Erfle, Biotechniques 2009, 47, 877-878.
[9.] J. Shiu, C. Kuo, W. Whanga, P. Chen, Lab on a Chip 2010, 10, 556-558.
[10.] WO 2009/137267 A2

## Claims

1. A patterned substrate with a transparent solid support coated with a porous polymer layer or film comprising
(i) hydrophilic areas; surrounded by
(ii) hydrophobic areas separating the hydrophilic areas into a predetermined spatial pattern;
wherein the porous polymer layer or film comprises porous poly(2-hydroxyethyl methacrylate-co-ethylene dimethacrylate) (HEMAcoEDMA) which has been copolymerized in the presence of 40 to 90 vol% of an inert porogen,
wherein the porous polymer layer or film has a thickness of 15 µm or less, and
wherein the hydrophobic areas are created by modifying the porous polymer layer or film.

2. The patterned substrate according to claim 1, wherein the hydrophilic areas are separated by the hydrophobic areas so as to form an array of hydrophilic spots surrounded by a hydrophobic porous polymer surface.

3. The patterned substrate according to claim 1 or 2, wherein the hydrophilic areas are separated by the hydrophobic areas of porous polymer surface so as to form hydrophilic channels.

4. The patterned substrate according to any of claims 1 to 3, further comprising (iii) at least one biologically active agent provided in at least one of the hydrophilic areas.

5. A method for the manufacture of the patterned substrate according to any of claims 1 to 4, comprising the steps of:
(a) providing a polymerization mixture between two transparent solid supports, wherein the polymerization mixture comprises 2-hydroxyethyl methacrylate (HEMA) and ethylene dimethacrylate (EDMA) in the presence of 40 to 90 vol% of an inert porogen;
(b) polymerizing the polymerization mixture by free radical polymerization to form a porous polymer layer having a thickness of 15 µm or less;
(c) removing one of the two transparent solid supports; and
(d) modifying the surface of the porous polymer layer, thereby creating a pattern of hydrophilic areas surrounded by hydrophobic areas.

6. The method according to claim 5, wherein the porous polymer layer possesses hydrophilicity and is prepared by radical polymerization of said polymerization mixture.

7. The method according to claim 5, wherein the hydrophobic pattern is produced by photografting of the hydrophilic porous polymer layer with hydrophobic mono- or poly-vinyl monomers or mixtures thereof.

8. The method according to any of claims 5 to 7, wherein the hydrophilic porous polymer surface comprises poly(2-hydroxyethyl methacrylate-co-ethylene dimethacrylate) and the photografted hydrophobic monomer is 2,2,3,3,3-pentafluoropropyl methacrylate.

9. The method according to any of claims 5 to 8, which further comprises step (e) providing at least one biologically active agent in at least one hydrophilic area of the pattern.

10. The method according to any of claims 5 to 9, which further comprises step (f) seeding cells to be cultivated into at least one superhydrophilic spot.

11. A method for creating predefined patterns of cells by cultivating them on a patterned substrate according to any of claims 1 to 4.

12. The method according to claim 11, wherein the pattern formed by cells is a cell array, wherein the cells are in addressable positions which are predefined by the patterned substrate.

13. A method for cultivating cells comprising the steps of:
(I) providing a patterned substrate according to any of claims 1 to 4;
(II) seeding cells to be cultivated into at least one hydrophilic area of the patterned substrate; and
(III) cultivating the seeded cells.

14. The method according to claim 13, wherein at least one hydrophilic spot of the patterned substrate provided in step (I) contains at least one biologically active agent.

15. Use of a patterned substrate according to any of claims 1 to 4 for cultivating mammalian or prokaryotic cells.

16. The use according to claim 15, wherein the patterned substrate further comprises
(iii) at least one biologically active agent provided in at least one hydrophilic areas.

## Patentansprüche

1. Strukturiertes Substrat mit einem transparenten festen Träger, beschichtet mit einer porösen Polymerschicht oder einem porösen Polymerfilm, umfassend
(i) hydrophile Bereiche, umgeben von
(ii) hydrophoben Bereichen, welche die hydrophilen Bereiche in eine vorbestimmte räumliche Struktur trennen,
wobei die poröse Polymerschicht oder der poröse Polymerfilm poröses Poly(2-Hydroxyethylmethacrylat-co-Ethylendimethacrylat) (HEMAcoEDMA), welches in der Gegenwart von 40 bis 90 Vol.-% eines inerten Porogens copolymerisiert wurde, umfasst,
wobei die poröse Polymerschicht oder der poröse Polymerfilm eine Dicke von 15 µm oder weniger aufweist und
wobei die hydrophoben Bereiche durch Modifizieren der porösen Polymerschicht oder des porösen Polymerfilms erzeugt sind.

2. Strukturiertes Substrat nach Anspruch 1, wobei die hydrophilen Bereiche durch die hydrophoben Bereiche getrennt sind, um so eine Anordnung von hydrophilen Stellen, umgeben von einer hydrophoben porösen Polymeroberfläche, zu bilden.

3. Strukturiertes Substrat nach Anspruch 1 oder 2, wobei die hydrophilen Bereiche durch die hydrophoben Bereiche einer porösen Polymeroberfläche getrennt sind, um so hydrophile Kanäle zu bilden.

4. Strukturiertes Substrat nach einem der Ansprüche 1 bis 3, weiter umfassend
(iii) mindestens einen biologisch aktiven Wirkstoff, bereitgestellt in mindestens einem der hydrophilen Bereiche.

5. Verfahren zur Herstellung des strukturierten Substrats nach einem der Ansprüche 1 bis 4, umfassend die Schritte:
(a) Bereitstellen eines Polymerisationsgemisches zwischen zwei transparenten festen Trägern, wobei das Polymerisationsgemisch 2-Hydroxyethylmethacrylat (HEMA) und Ethylendimethacrylat (EDMA) in der Gegenwart von 40 bis 90 Vol.-% eines inerten Porogens umfasst,
(b) Polymerisieren des Polymerisationsgemisches durch freie radikalische Polymerisation, um eine poröse Polymerschicht mit einer Dicke von 15 µm oder weniger zu bilden,
(c) Entfernen eines der beiden transparenten festen Träger und
(d) Modifizieren der Oberfläche der porösen Polymerschicht, wodurch eine Struktur von hydrophilen Bereichen, umgeben von hydrophoben Bereichen, erzeugt wird.

6. Verfahren nach Anspruch 5, wobei die poröse Polymerschicht Hydrophilizität aufweist und durch radikalische Polymerisation des Polymerisationsgemisches hergestellt wird.

7. Verfahren nach Anspruch 5, wobei die hydrophobe Struktur durch Photopfropfen der hydrophilen porösen Polymerschicht mit hydrophoben Mono- oder Polyvinylmonomeren oder Gemischen davon hergestellt wird.

8. Verfahren nach einem der Ansprüche 5 bis 7, wobei die hydrophile poröse Polymeroberfläche Poly(2-Hydroxyethylmethacrylat-co-Ethylendimethacrylat) umfasst und das photogepfropfte hydrophobe Monomer 2,2,3,3,3-Pentafluorpropylmethacrylat ist.

9. Verfahren nach einem der Ansprüche 5 bis 8, welches weiter einen Schritt (e) des Bereitstellens von mindestens einem biologisch aktiven Wirkstoff in mindestens einem hydrophilen Bereich der Struktur umfasst.

10. Verfahren nach einem der Ansprüche 5 bis 9, welches weiter einen Schritt (f) des Aussäens von zu kultivierenden Zellen in mindestens eine superhydrophile Stelle umfasst.

11. Verfahren zum Erzeugen vordefinierter Strukturen von Zellen, indem diese auf einem strukturierten Substrat nach einem der Ansprüche 1 bis 4 kultiviert werden.

12. Verfahren nach Anspruch 11, wobei die durch Zellen gebildete Struktur eine Zellanordnung ist, wobei sich die Zellen in adressierbaren Positionen, welche durch das strukturierte Substrat vordefiniert sind, befinden.

13. Verfahren zum Kultivieren von Zellen, umfassend die Schritte:
(I) Bereitstellen eines strukturierten Substrats nach einem der Ansprüche 1 bis 4,
(II) Aussäen von zu kultivierenden Zellen in mindestens einen hydrophilen Bereich des strukturierten Substrats und
(III) Kultivieren der ausgesäten Zellen.

14. Verfahren nach Anspruch 13, wobei mindestens eine hydrophile Stelle des strukturierten Substrats, bereitgestellt in Schritt (I), mindestens einen biologisch aktiven Wirkstoff enthält.

15. Verwendung eines strukturierten Substrats nach einem der Ansprüche 1 bis 4 zum Kultivieren von Säuger- oder prokaryotischen Zellen.

16. Verwendung nach Anspruch 15, wobei das strukturierte Substrat weiter
(iii) mindestens einen biologisch aktiven Wirkstoff, bereitgestellt in mindestens einem der hydrophilen Bereiche, umfasst.

## Revendications

1. Substrat à motifs doté d'un support solide transparent revêtu d'une couche ou d'un film polymère poreux comprenant :
(i) des zones hydrophiles ; entourées par :
(ii) des zones hydrophobes séparant les zones hydrophiles en un motif spatial prédéterminé ;
dans lequel la couche ou le film polymère poreux comprend du poly(méthacrylate de 2-hydroxyéthyle-co-diméthacrylate d'éthylène) poreux (HEMAcoEDMA) qui a été copolymérisé en présence de 40 à 90 % en volume d'un porogène inerte,
dans lequel la couche ou le film polymère poreux a une épaisseur de 15 µm ou moins, et dans lequel les zones hydrophobes sont créées en modifiant la couche ou le film polymère poreux.

2. Substrat à motifs selon la revendication 1, dans lequel les zones hydrophiles sont séparées par les zones hydrophobes de façon à former un réseau de taches hydrophiles entourées par une surface de polymère poreux hydrophobe.

3. Substrat à motifs selon la revendication 1 ou 2, dans lequel les zones hydrophiles sont séparées par les zones hydrophobes de surface de polymère poreux de manière à former des canaux hydrophiles.

4. Substrat à motifs selon l'une quelconque des revendications 1 à 3, comprenant en outre :
(iii) au moins un agent biologiquement actif fourni dans au moins l'une des zones hydrophiles.

5. Procédé de fabrication du substrat à motifs selon l'une quelconque des revendications 1 à 4, comprenant les étapes de :
(a) fourniture d'un mélange de polymérisation entre deux supports solides transparents, dans lequel le mélange de polymérisation comprend du méthacrylate de 2-hydroxyéthyle (HEMA) et du diméthacrylate d'éthylène (EDMA) en présence de 40 à 90 % en volume d'un porogène inerte ;
(b) polymérisation du mélange de polymérisation par polymérisation par radicaux libres pour former une couche de polymère poreux ayant une épaisseur de 15 µm ou moins ;
(c) élimination de l'un des deux supports solides transparents ; et
(d) modification de la surface de la couche de polymère poreux, créant ainsi un motif de zones hydrophiles entourées par des zones hydrophobes.

6. Procédé selon la revendication 5, dans lequel la couche de polymère poreux possède une hydrophilie et est préparée par polymérisation par radicaux dudit mélange de polymérisation.

7. Procédé selon la revendication 5, dans lequel le motif hydrophobe est produit par photogreffage de la couche de polymère poreux hydrophile avec des monomères mono- ou poly-vinyle hydrophobes ou leurs mélanges.

8. Procédé selon l'une quelconque des revendications 5 à 7, dans lequel la surface de polymère poreux hydrophile comprend du poly(méthacrylate de 2-hydroxyéthyle-co-diméthacrylate d'éthylène) et le monomère hydrophobe photogreffé est le méthacrylate de 2,2,3,3,3-pentafluoropropyle.

9. Procédé selon l'une quelconque des revendications 5 à 8, qui comprend en outre l'étape (e) de fourniture d'au moins un agent biologiquement actif dans au moins une zone hydrophile du motif.

10. Procédé selon l'une quelconque des revendications 5 à 9, qui comprend en outre l'étape (f) d'ensemencement de cellules à cultiver dans au moins une tache superhydrophile.

11. Procédé de création de motifs prédéfinis de cellules par leur culture sur un substrat à motifs selon l'une quelconque des revendications 1 à 4.

12. Procédé selon la revendication 11, dans lequel le motif formé par des cellules est un réseau de cellules, dans lequel les cellules sont dans des positions adressables qui sont prédéfinies par le substrat à motifs.

13. Procédé de culture de cellules comprenant les étapes de :
(I) fourniture d'un substrat à motifs selon l'une quelconque des revendications 1 à 4 ;
(II) ensemencement de cellules à cultiver dans au moins une zone hydrophile du substrat à motifs ; et
(III) culture de cellules ensemencées.

14. Procédé selon la revendication 13, dans lequel au moins une tache hydrophile du substrat à motifs fourni à l'étape (I) contient au moins un agent biologiquement actif.

15. Utilisation d'un substrat à motifs selon l'une quelconque des revendications 1 à 4, pour cultiver des cellules mammaliennes ou procaryotes.

16. Utilisation selon la revendication 15, dans laquelle le substrat à motifs comprend en outre :
(iii) au moins un agent biologiquement actif fourni dans au moins une zone hydrophile.
